# FASCICULE DE BREVET EUROPEEN

(11) **EP 3 405 544 B1**
(45) Date de publication et mention de la délivrance du brevet: **19.05.2021**
(21) Numéro de dépôt: 17706548.9
(22) Date de dépôt: 19.01.2017
(51) Int. Cl.: C09K 5/06, A61F 7/02, F25D 3/08, A61L 15/00, A61L 26/00

(54) **COMPOSITION CRYOGENIQUE**
KRYOGENE ZUSAMMENSETZUNG
CRYOGENIC COMPOSITION

(30) Priorité: 20.01.2016 FR 1650448
(43) Date de publication de la demande: 28.11.2018
(73) Titulaire: Lesage, Patrick, 35400 Saint Malo (FR); Lesage, Alexandre, 35000 Rennes (FR)
(72) Inventeur: Lesage, Patrick, 35400 Saint Malo (FR); Lesage, Alexandre, 35000 Rennes (FR)
(74) Mandataire: Cabinet Beau de Loménie
(86) Numéro de dépôt international: PCT/FR2017/050113
(87) Numéro de publication internationale: WO 2017/125687

(56) Documents cités:
- US-A- 5 513 629
- US-A- 5 800 491
- US-A1- 2002 042 641
- US-A1- 2009 005 842
- US-A1- 2011 024 674
- US-B1- 6 379 582
- Anonymous: "Squalane - Wikipedia", , 20 March 2020 (2020-03-20), XP055680115, Retrieved from the Internet: URL:https://en.wikipedia.org/wiki/Squalane [retrieved on 2020-03-26]

## Description

La présente invention concerne une nouvelle composition cryogénique destinée à être utilisée dans des dispositifs de traitement thermique, ainsi que son procédé de préparation. L'invention concerne également un dispositif de traitement thermique, et plus particulièrement un dispositif médical utilisé pour refroidir une partie du corps humain ou animal, en particulier après un traumatisme, une inflammation ou un acte chirurgical. L'invention trouve donc application dans le domaine thérapeutique et/ou médical, mais également dans d'autres domaines comme par exemple pour le refroidissement ou le maintien à basse température de denrées alimentaires.

La cryothérapie ou traitement par le froid a fait son apparition dans les années 1970, et est aujourd'hui largement utilisée dans le domaine médical pour guérir les tissus lésés, favoriser la cicatrisation, soulager des douleurs corporelles telles que des entorses, tendinites, claquages musculaires. Son principe consiste à exposer au froid une partie du corps humain ou animal pour l'amener à secréter des endorphines, ce qui a pour effet de provoquer une analgésie, et donc un abaissement du seuil de la douleur. La cryothérapie est ainsi utilisée après des actes post-traumatiques ou post-chirurgicaux, pour son action hémolytique et anti-œdémateuse.

La cryothérapie utilise différentes sources de froid :
- Des dispositifs à base d'azote provenant de la gazéification instantanée d'azote liquide, cette technique impliquant l'utilisation d'un matériel lourd et encombrant, et par conséquent peu maniable.
- Des dispositifs de traitement thermique à base d'eau. L'eau est un des matériaux qui restitue le plus le froid. Toutefois, à une température inférieure à 0°C, l'eau se transforme en glace et présente certains inconvénients du fait de sa structure solide difficilement conformable à une structure anatomique, et dont la très basse température provoque des lésions des tissus.
- Des dispositifs à base de compositions antigel ne présentant pas de changement de phase lors de la congélation à -25°C. Ces compositions, présentes sous forme de billes ou de gel, restituent un froid très instable ne permettant pas une utilisation effective en cryothérapie. Les dispositifs utilisant ces compositions présentent lors de la décongélation une température de surface qui peut être de l'ordre de -5°C pour les gels, à 10°C pour les billes.
- Des dispositifs à changement de phase tels que ceux décrits dans le brevet EP 1 011 558. Ce brevet décrit des compositions comprenant de l'eau, un produit absorbant de type polymère acrylique et un agent humectant. Lors de la congélation, une partie de l'eau contenue dans le réseau du produit absorbant est libérée et change de phase pour se transformer en cristaux de glace. Lors du réchauffement, l'eau réintègre le réseau absorbant. Ces compositions restent souples et conformables. Elles sont cependant peu résistantes aux cycles répétés de congélation/décongélation, et ont tendance à devenir de moins en moins souples au fur et à mesure des cycles, ce qui les rend inconfortables et moins efficaces à l'usage. Ces compositions présentent en outre l'inconvénient de nécessiter un contenant comportant une zone semi-perméable étanche à l'eau et perméable à l'air, ce qui complexifie le dispositif. Ce dernier présente aussi, lors de la décongélation, une température de surface inférieure à 0°C pendant une longue période, ce qui diminue d'autant la durée d'utilisation du dispositif car il convient d'attendre que la température se stabilise au-dessus de 0°C ou d'intercaler une couche isolante entre le dispositif et la peau pour l'utiliser.

Le document US 2011/024674 A1 (D1) décrit une composition de refroidissement utilisée entre autre dans des vêtements techniques ou bien à usage médical.

Le document US 5,513,629 A divulgue une composition susceptible d'être chauffée ou congelée.

Le document US 2009/005842 A1 décrit une couverture de refroidissement d'une partie du corps d'un mammifère.

Dans ce contexte, la présente invention a pour but de remédier aux inconvénients des dispositifs de l'art antérieur en proposant un nouveau dispositif de traitement thermique souple et étanche qui reste parfaitement souple après congélation, et ne s'altère pas rapidement au cours des cycles de congélation/décongélation. Le dispositif de l'invention présente l'avantage de pouvoir être réutilisé plusieurs fois (jusqu'à une trentaine de fois). Quelques minutes après sa décongélation, le dispositif de l'invention présente une température de surface légèrement supérieure à 0°C. Cette température, idéalement entre 3 et 5°C, reste stable pendant une durée d'au moins 60 minutes, et pouvant aller jusqu'à 120 minutes.

Les Inventeurs ont découvert qu'il était possible d'atteindre ces performances en utilisant une composition cryogénique à base de polymère superabsorbant, d'eau et d'agent humectant, incorporée dans un composé hydrophobe. Ils ont observé que le composé hydrophobe crée un film hydrophobe ou « couche isolante » autour des granulés de polymère, emprisonnant ainsi une partie de l'eau en leur sein. Cette propriété confère une très grande souplesse à la composition cryogénique.

Ainsi, selon un premier aspect, la présente invention a pour objet une composition cryogénique comprenant un composé hydrophobe (a) sous forme liquide dans lequel sont immergés des granulés de polymère superabsorbant (b) chargés en eau (c) et en agent humectant (d), ledit composé hydrophobe (a) ayant un point de congélation inférieur à -10°C et étant choisi parmi le diheptanoate de néopentylène glycol, le sébacate d'isopropyl, le néopentanoate d'isodécyle, l'isostéaryl isostéarate, et leurs mélanges.

Le composé hydrophobe (a) susceptible d'être utilisé dans le cadre de l'invention présente avantageusement un point de congélation inférieur à -15°C. De manière particulièrement préférée, le composé hydrophobe (a) présente un point de congélation inférieur à -20°C. Il présente avantageusement un haut poids moléculaire, allant de 200 à 700 g.mol⁻¹. Les composés hydrophobes (a) choisis sont commercialisés par exemple par la société STÉARINERIE DUBOIS sous les références DUB DNPG (diheptanoate de néopentylène glycol, point de congélation : -55°C), DUB DIS (sébacate d'isopropyl, point de congélation : - 20°C), DUB VCI 10 (néopentanoate d'isodécyle, point de congélation : -35°C), DUB ISIS (isostéaryl isostéarate).

Le composé hydrophobe (a) représente de préférence de 0,2 à 3% en poids, et encore plus préférentiellement de 0,2 à 2%, rapporté au poids total de la composition cryogénique.

On entend par « polymère superabsorbant » un polymère qui est apte à son état sec à absorber spontanément au moins 20 fois son propre poids de fluide aqueux, en particulier d'eau. Ce polymère a une grande capacité d'absorption et de rétention de l'eau et de fluides aqueux. Après absorption du liquide aqueux, les particules de polymère ainsi imbibée de fluide aqueux restent insolubles dans le fluide aqueux, et conservent ainsi leur état particulaire individualisé. Des exemples de polymères superabsorbants sont décrits dans l'ouvrage « Absorbent polymer technology, Studies in polymer science 8 » de L. BRANNON-PAPPAS et R. HARLAND, édition Elsevier, 1990.

Parmi les polymères superabsorbants (b) susceptibles d'être utilisés dans le cadre de la présente invention, on peut citer les polyacrylates de sodium ou de potassium réticulés, les polyacrylamides, les copolymères à base d'éthylène et d'anhydride maléique, les copolymères d'alcool vinylique, l'oxyde de polyéthylène réticulé, les polymères à base d'amidon, de gomme et de dérivés cellulosiques, les pectines, les alginates, l'agar-agar (ou agarose), les polyéthylène amines, les polyvinyle amines et leurs mélanges. Le polymère superabsorbant (b) est de préférence un homo- ou copolymère acrylique réticulé, et encore plus préférentiellement un homo- ou copolymère acrylique de potassium réticulé.

Le polymère superabsorbant (b) est généralement utilisé en une quantité allant de 1 à 6% en poids, et de préférence de 1,5 à 3%, rapporté au poids total de la composition cryogénique.

De manière avantageuse, les granulés de polymère superabsorbant (b) de l'invention se présentent sous forme de particules sphériques ayant un diamètre de 1 à 6 mm lorsqu'ils sont déshydratés. Selon un mode de réalisation encore plus préféré, les particules sphériques de polymère superabsorbant (b) sont des billes de polyacrylate de sodium ou de potassium, et encore plus préférentiellement de polyacrylate de potassium, ayant de préférence un diamètre de 1 à 3 mm lorsqu'elles sont déshydratées. Lesdites particules sphériques peuvent absorber plus de 40 fois leur masse, cette caractéristique étant définie dans des conditions normales de température (25°C) et de pression (100000 Pa) et pour de l'eau. Une fois hydratée, les particules sphériques de polymère superabsorbant (b) gonflent et forment des billes molles.

L'agent humectant (d) de l'invention peut être choisi parmi le glycérol, le sorbitol, le polyéthylène glycol, le (di)propylène glycol, le polypropylène glycol, le 1,5-pentanediol, le propylène glycol, le butylène glycol, le diéthylène glycol, l'huile de paraffine et leurs mélanges, de préférence parmi le glycérol, le (di)propylène glycol, le polypropylène glycol et leurs mélanges, et encore plus préférentiellement parmi le (di)propylène glycol, le polypropylène glycol et leurs mélanges. Le dipropylène glycol est l'agent humectant (d) le plus préféré.

L'agent humectant (d) est généralement utilisé en une quantité allant de 6 à 10% en poids, et de préférence de 7 à 9%, rapporté au poids total de la composition cryogénique.

Selon un mode de réalisation préféré, la composition cryogénique de l'invention comprend :
(a) de 0,2 à 3%, et de préférence de 0,2 à 2%, en poids du composé hydrophobe,
(b) de 1 à 6%, et de préférence de 1,5 à 3%, en poids de granulés de polymère superabsorbant,
(c) de 75 à 95%, et de préférence de 85 à 90%, en poids d'eau ;
(d) de 6 à 10%, et de préférence de 7 à 9%, en poids d'un agent humectant,
lesdits pourcentages étant exprimés en pourcentage en poids par rapport au poids total de la composition, et le poids total de la composition représentant 100%.

Un autre objet concerne un procédé de préparation d'une composition cryogénique selon l'invention comprenant les étapes suivantes :
(i) mélange de l'eau (c) et de l'agent humectant (d), sous agitation,
(ii) ajout des granulés de polymère superabsorbant (b) dans le mélange obtenu à l'issu de l'étape (i), sous agitation,
(iii) maturation des granulés de polymère superabsorbant (b) présents au sein du mélange obtenu à l'issu de l'étape (ii), en laissant reposer ledit mélange à température ambiante, de préférence jusqu'à ce que les granulés de polymère superabsorbant (b) aient atteint une taille comprise entre deux et quatre fois leur taille initiale,
(iv) incorporation du mélange obtenu à l'issu de l'étape (iii) dans un contenant comprenant le composé hydrophobe (a) sous forme liquide, sous agitation, afin de créer un film hydrophobe ou « couche isolante » autour des granulés de polymère superabsorbant (b).

L'étape (iii) de maturation des granulés de polymère superabsorbant (b) est de préférence réalisée pendant une durée allant de 1 à 5 heures, et encore plus préférentiellement pendant une durée allant de 2 à 4 heures.

Un dispositif de traitement thermique comprenant un contenant étanche, à l'intérieur duquel est contenue la présente composition cryogénique, fait également partie de l'invention. Le contenant est avantageusement constitué en un matériau souple tel que le polychlorure de vinyle (PVC), le polychloroprène (néoprène), le polytétrafluoroéthylène (PTFE) ou le polyéthylène (PE), et de préférence en polychlorure de vinyle (PVC). Le dispositif est de préférence une poche médicale étanche et flexible, qui peut être appliquée sur une partie du corps humain ou animal, par exemple pour résorber les hématomes, les œdèmes, ou pour calmer la douleur.

L'invention vise donc principalement des dispositifs médicaux choisis parmi un masque facial, une attelle pour l'épaule, le coude, la cheville, le genou, la hanche ou le poignet, et tout support comprenant une poche médicale étanche et flexible selon l'invention.

La composition cryogénique selon l'invention, ou le dispositif de traitement thermique selon l'invention, peut être utilisé pour refroidir une partie du corps humain ou animal, de préférence après un traumatisme, une inflammation ou un acte chirurgical. La température peut être contrôlée visuellement à l'aide d'un pigment thermochrome, préalablement ajouté à la composition cryogénique de l'invention. Il peut également être contenu dans le matériau constituant le contenant. Ce pigment peut être un pigment thermochrome tel que ceux commercialisés par la société OliKrom.

Le dispositif de traitement thermique de l'invention peut aussi être destiné à des applications non médicales, et être utilisé pour abaisser ou maintenir la température de denrées alimentaires, ou pour favoriser leur conservation, ou pour le transport en ambiance froide d'articles sensibles à la chaleur. Dans ce cas, la température peut être contrôlée visuellement à l'aide d'un pigment thermochrome tel que décrit précédemment.

Le dispositif de traitement thermique de l'invention peut être utilisé dans un procédé de traitement thermique, de préférence d'une partie du corps humain ou animal, comprenant les étapes suivantes :
(i') congélation d'un dispositif selon l'invention à une température inférieure à 0°C, et de préférence à une température comprise entre -20°C et - 30°C, pendant une durée pouvant aller de 2 à 4 heures,
(ii') malaxation manuelle du dispositif obtenu à l'issu de l'étape (i'),
(iii') application du dispositif obtenu à l'issu de l'étape (ii') sur une partie du corps humain ou animal, à l'aide ou non d'un dispositif médical selon l'invention, de préférence pendant une durée comprise entre 30 minutes et 120 minutes.

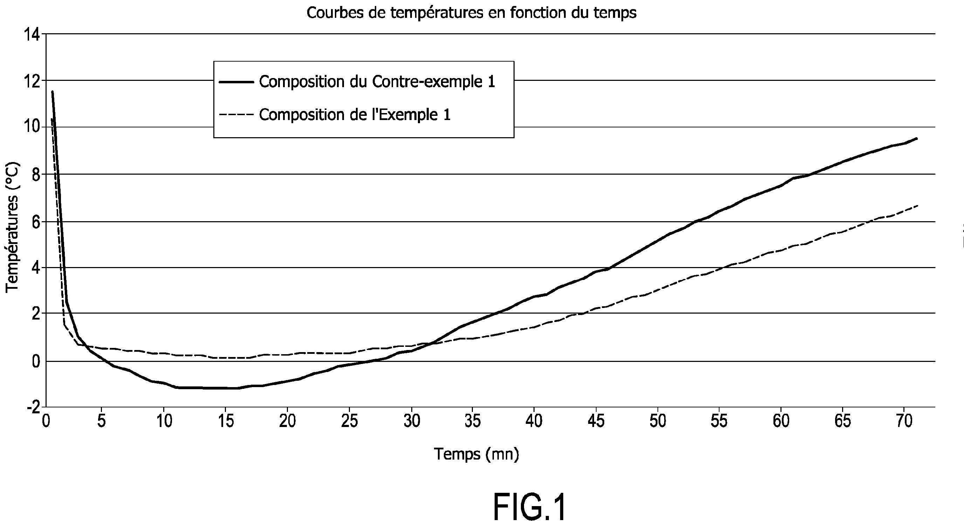

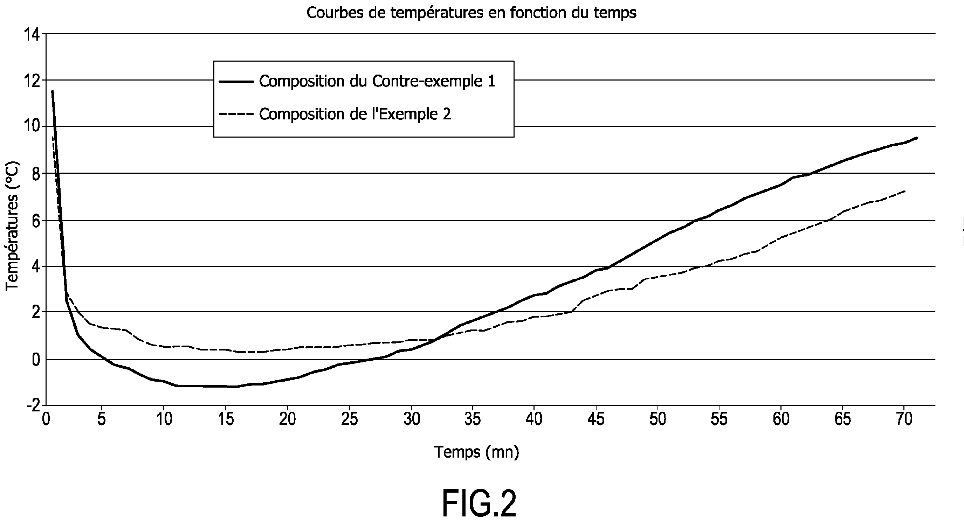

Outre les dispositions qui précèdent, l'invention comprend encore d'autres dispositions qui ressortiront du complément de description qui suit, qui se rapporte à des exemples mettant en évidence les propriétés avantageuses de la composition de l'invention, ainsi qu'aux graphiques des **Figures 1** et **2** (FIG. 1 et 2) annexées qui représentent les courbes de températures en fonction du temps obtenues pour les compositions cryogéniques de l'Exemple 1 et du Contre-exemple 1, et de l'Exemple 2.

### EXEMPLES :

### Exemple 1 :

Une composition selon l'invention est préparée en mélangeant 26,3 g d'eau avec 2,24 g de dipropylène glycol (fournisseur : INTERCHIMIE), sous agitation manuelle. 0,65 g de billes de polyacrylate de potassium ayant un diamètre de 1,5 mm (hydro perles Floragel, fournisseur : AXEVI) sont ajoutées au mélange eau et dipropylène glycol. Le mélange est laissé au repos à température ambiante pendant 3 heures (étape d'hydratation des billes de polymère), jusqu'à ce que les billes de polyacrylate de potassium aient atteints un diamètre moyen de 5,5 mm. 0,43 g de diheptanoate de néopentylène glycol ayant un point de congélation de -55°C (DUB DPNG de la STÉARINERIE DUBOIS) est versé dans une poche en PVC de 5 ^{∗} 7 cm et de 0,3 mm d'épaisseur. Le mélange à base d'eau, de dipropylène glycol et de billes de polyacrylate de potassium, préalablement préparé, est également ajouté dans la poche en PVC. La poche est ensuite scellée et placée au congélateur à -20°C pendant 3 heures. Lors de la congélation une partie de l'eau contenue dans les billes de polymère change de phase et se transforme en petits cristaux de glace ayant l'apparence de la neige. La poche en PVC reste par ailleurs souple et conformable à l'issu de l'étape de congélation.

La température de l'échantillon est mesurée chaque minute pendant 70 minutes sous une compression de 14 g.cm⁻², grâce à un enregistreur de température TESTO 175 T2, avec une sonde de type CTN. Les mesures sont réalisées toutes les minutes à l'interface entre la poche congelée et une poche d'eau à 20°C de 2 cm d'épaisseur. La courbe de température en fonction du temps est représentée sur la **Figure 1** (FIG. 1). La température de la poche reste toujours au-dessus de 0°C, et se maintient en-dessous de 5°C pendant 61 minutes. La poche est donc utilisable pour un traitement par cryothérapie pendant 61 minutes.

Le vieillissement de la composition est également évalué par mesure du nombre de cycles de congélation/décongélation. Le vieillissement devient visible lorsque les billes de polymère commencent à s'agréger, conduisant à une diminution de la souplesse de la poche congelée. Avec la composition de l'invention de l'Exemple 1, la poche reste homogène et souple jusqu'au 25^{ème} cycle de congélation/décongélation.

### Exemple 2 :

Une composition selon l'invention est préparée en mélangeant 1000 g d'eau avec 10 g de butylène glycol, 10 g de pentylène glycol, 10 g de propylène glycol et 50 g de de dipropylène glycol (fournisseur : INTERCHIMIE), sous agitation manuelle. 25 g de billes de polyacrylate de potassium ayant un diamètre de 3 mm (hydro perles Floragel, fournisseur : AXEVI) sont ajoutées au mélange eau, de butylène glycol, de pentylène glycol, de propylène glycol et de dipropylène glycol. Le mélange est laissé au repos à température ambiante pendant 3 heures (étape d'hydratation des billes de polymère), jusqu'à ce que les billes de polyacrylate de potassium aient atteints un diamètre moyen de 8 mm. 5 g de néopentanoate d'isodécyle ayant un point de congélation de -35°C (DUB VCI 10 de la STÉARINERIE DUBOIS) est versé dans une poche en PVC de 5 ^{∗} 7 cm et de 0,3 mm d'épaisseur. Le mélange à base d'eau, de butylène glycol, de pentylène glycol, de propylène glycol, de dipropylène glycol et de billes de polyacrylate de potassium, préalablement préparé, est également ajouté dans la poche en PVC. La poche est ensuite scellée et placée au congélateur à -20°C pendant 3 heures. Lors de la congélation une partie de l'eau contenue dans les billes de polymère change de phase et se transforme en petits cristaux de glace ayant l'apparence de la neige.

La température de l'échantillon est mesurée chaque minute pendant 70 minutes sous une compression de 14 g.cm⁻², grâce à un enregistreur de température TESTO 175 T2, avec une sonde de type CTN. Les mesures sont réalisées toutes les minutes à l'interface entre la poche congelée et une poche d'eau à 20°C de 2 cm d'épaisseur. La courbe de température en fonction du temps est représentée sur la **Figure 2** (FIG. 2). La température de la poche reste toujours au-dessus de 0°C, et se maintient en-dessous de 5°C pendant 58 minutes. La poche est donc utilisable pour un traitement par cryothérapie pendant 58 minutes.

Le vieillissement de la composition est également évalué par mesure du nombre de cycles de congélation/décongélation. Le vieillissement devient visible lorsque les billes de polymère commencent à s'agréger, conduisant à une diminution de la souplesse de la poche congelée. Avec la composition de l'invention de l'Exemple 2, la poche reste homogène et souple jusqu'au 25^{ème} cycle de congélation/décongélation.

### Contre-exemple 1 :

Une composition selon l'exemple 1 du brevet EP 1 011 558 est préparée en mélangeant 27,75 g d'eau avec 1,80 g de propylène glycol, sous agitation manuelle. 0,45 g de billes de copolymère acrylamide TERRA-SORB ayant un diamètre compris entre 1 et 6 mm (fournisseur : Plant Health Care) sont ajoutées au mélange eau et propylène glycol. Le mélange est laissé au repos à température ambiante pendant 3 heures (étape d'hydratation des billes de polymère), jusqu'à ce que les billes de polyacrylate de potassium aient atteints un diamètre moyen de 8 à 9 mm. La composition ainsi préparée est placée dans une poche en PVC de 5 ^{∗} 7 cm et de 0,3 mm d'épaisseur. La poche est ensuite scellée et placée au congélateur à -20°C pendant 3 heures.

Comme pour l'Exemple 1, la température de l'échantillon est mesurée chaque minute pendant 70 minutes sous une compression de 14 g.cm⁻², grâce à un enregistreur de température TESTO 175 T2, avec une sonde de type CTN. Les mesures sont réalisées toutes les minutes à l'interface entre la poche congelée et une poche d'eau à 20°C de 2 cm d'épaisseur. La courbe de température en fonction du temps est également représentée sur les **Figures 1** et **2** (FIG. 1 et 2), pour comparaison avec les compositions de l'invention des Exemples 1 et 2. La température de la poche reste inférieure à 0°C pendant environ 25 minutes, puis se maintient entre 0 et 5°C pendant 24 minutes. La poche est donc utilisable pour un traitement par cryothérapie pendant 24 minutes.

Comme pour les Exemples 1 et 2, le vieillissement de la composition est évalué par mesure du nombre de cycles de congélation/décongélation. Dès le 1^{er} cycle, il est observé que la poche est moins homogène et moins souple que les poches des Exemples 1 et 2. Avec la composition du brevet EP 1 011 558, la poche reste homogène et souple jusqu'au 10^{ème} cycle de congélation/décongélation. A partir du 11^{ème} cycle, on observe une agrégation des billes de polymère.

### Contre-exemple 2 :

Une composition analogue à celle de l'Exemple 1 ci-dessus est préparée, mais sans diheptanoate de néopentylène glycol (absence de composé hydrophobe dans la composition).

La poche congelée obtenue est moins souple que celle de l'Exemple 1 contenant le composé hydrophobe.

Comme pour l'Exemple 1, le vieillissement de la composition est évalué par mesure du nombre de cycles de congélation/décongélation. En l'absence de composé hydrophobe, la composition présente un vieillissement prématuré à partir du 15^{ème} cycle de congélation/décongélation.

### Contre-exemple 3 :

Une composition selon l'invention est préparée comme décrit à l'Exemple 1 en remplaçant le diheptanoate de néopentylène glycol ayant un point de congélation de -55°C (DUB DPNG de la STÉARINERIE DUBOIS) par de l'huile de vaseline ayant un point de congélation de -10°C (HUILE DE VASELINE CODEX 22 de la société INTERCHIMIE). Le mélange ainsi préparé est ajouté dans la poche en PVC. La poche est ensuite scellée et placée au congélateur à -20°C pendant 3 heures. Lors de la congélation, la poche en PVC se rigidifie : on observe des interactions négatives entre l'huile de vaseline et la poche en PVC. La poche n'est pas suffisamment souple pour pouvoir être utilisée comme poche médicale flexible.

## Revendications

1. Composition cryogénique, **caractérisée en ce qu'**elle comprend un composé hydrophobe (a) sous forme liquide dans lequel sont immergés des granulés de polymère superabsorbant (b) chargés en eau (c) et en agent humectant (d), ledit composé hydrophobe (a) ayant un point de congélation inférieur à -10°C et étant choisi parmi le diheptanoate de néopentylène glycol, le sébacate d'isopropyl, le néopentanoate d'isodécyle, l'isostéaryl isostéarate, et leurs mélanges.

2. Composition cryogénique selon la revendication 1, **caractérisée en ce qu'**elle comprend :
(a) de 0,2 à 3%, et de préférence de 0,2 à 2%, en poids d'un composé hydrophobe dont le point de congélation est inférieur à 0°C,
(b) de 1 à 6%, et de préférence de 1,5 à 3%, en poids de granulés de polymère superabsorbant,
(c) de 75 à 95%, et de préférence de 85 à 90%, en poids d'eau ;
(d) de 6 à 10%, et de préférence de 7 à 9%, en poids d'un agent humectant,
lesdits pourcentages étant exprimés en pourcentage en poids par rapport au poids total de la composition, et le poids total de la composition représentant 100%.

3. Composition cryogénique selon la revendication 1 ou la revendication 2, **caractérisée en ce que** le composé hydrophobe (a) a un point de congélation inférieur à -15°C, et encore plus préférentiellement inférieur à -20°C.

4. Composition cryogénique selon l'une des revendications 1 à 3, **caractérisée en ce que** le polymère superabsorbant (b) est choisi parmi les polyacrylates de sodium ou de potassium réticulés, les polyacrylamides, les copolymères à base d'éthylène et d'anhydride maléique, les copolymères d'alcool vinylique, l'oxyde de polyéthylène réticulé, les polymères à base d'amidon, de gomme et de dérivés cellulosiques, les pectines, les alginates, l'agar-agar (ou agarose), les polyéthylène amines, les polyvinyle amines et leurs mélanges.

5. Composition cryogénique selon l'une des revendications 1 à 4, **caractérisée en ce que** lesdits granulés de polymère superabsorbant (b) se présentent sous forme de particules sphériques ayant un diamètre de 1 à 6 mm lorsqu'ils sont déshydratées, lesdites particules sphériques pouvant absorber au moins 40 fois leur masse.

6. Composition cryogénique selon la revendication 5, **caractérisée en ce que** lesdites particules sphériques de polymère superabsorbant (b) sont des billes de polyacrylate de sodium ou de potassium, avantageusement de polyacrylate de potassium, ayant un diamètre de 1 à 3 mm lorsqu'elles sont déshydratées.

7. Composition cryogénique selon l'une des revendications 1 à 6, **caractérisée en ce que** l'agent humectant (d) est choisi parmi le glycérol, le sorbitol, le polyéthylène glycol, le (di)propylène glycol, le polypropylène glycol, le 1,5-pentanediol, le propylène glycol, le butylène glycol, le diéthylène glycol, l'huile de paraffine et leurs mélanges.

8. Composition cryogénique selon la revendication 7, **caractérisée en ce que** l'agent humectant (d) est le dipropylène glycol.

9. Procédé de préparation d'une composition cryogénique selon l'une des revendications 1 à 8, **caractérisé en ce qu'**il comprend les étapes suivantes :
(i) mélange de l'eau (c) et de l'agent humectant (d), sous agitation,
(ii) ajout des granulés de polymère superabsorbant (b) dans le mélange obtenu à l'issu de l'étape (i), sous agitation,
(iii) maturation des granulés de polymère superabsorbant (b) présents au sein du mélange obtenu à l'issu de l'étape (ii), en laissant reposer ledit mélange à température ambiante, de préférence jusqu'à ce que les granulés de polymère superabsorbant (b) aient atteint une taille comprise entre deux et quatre fois leur taille initiale,
(iv) incorporation du mélange obtenu à l'issu de l'étape (iii) dans un contenant comprenant le composé hydrophobe (a) sous forme liquide, sous agitation.

10. Procédé selon la revendication 9, **caractérisé en ce que** l'étape (iii) de maturation des granulés de polymère superabsorbant (b) est réalisée pendant une durée allant de 1 à 5 heures, et de préférence pendant une durée allant de 2 à 4 heures.

11. Dispositif de traitement thermique, **caractérisé en ce qu'**il comprend un contenant étanche, à l'intérieur duquel est contenu une composition cryogénique telle que définie aux revendications 1 à 8.

12. Dispositif selon la revendication 11, **caractérisé en ce que** ledit contenant est une poche médicale étanche et flexible constituée en un matériau choisi parmi le polychlorure de vinyle (PVC), le polychloroprène (néoprène), le polytétrafluoroéthylène (PTFE) ou le polyéthylène (PE).

13. Dispositif médical choisi parmi un masque facial, une attelle pour l'épaule, le coude, la cheville, le genou, la hanche ou le poignet, et tout support comprenant une poche médicale étanche et flexible telle que définie à la revendication 12.

14. Utilisation d'une composition cryogénique telle que définie selon l'une des revendications 1 à 8, ou d'un dispositif de traitement thermique selon la revendication 11, pour abaisser ou maintenir la température de denrées alimentaires, ou pour favoriser leur conservation, ou pour le transport en ambiance froide d'articles sensibles à la chaleur.

## Patentansprüche

1. Kryogene Zusammensetzung, **dadurch gekennzeichnet, dass** sie eine hydrophobe Verbindung (a) in flüssiger Form umfasst, in die mit Wasser (c) und Feuchthaltemittel (d) beladene superabsorbierende Polymergranulate (b) eingetaucht sind, wobei die hydrophobe Verbindung (a) einen Gefrierpunkt unter -10 ºC aufweist und aus Neopentenylglykol-Diheptanoat, Isopropylsebacat, Isodecylneopentanoat, Isostearylisostearat und deren Mischungen ausgewählt ist.

2. Kryogene Zusammensetzung nach Anspruch 1, **dadurch gekennzeichnet, dass** sie umfasst:
(a) 0,2 bis 3 Gew.-% und vorzugsweise 0,2 bis 2 Gew.-% einer hydrophoben Verbindung, deren Gefrierpunkt unter 0 ºC liegt,
(b) 1 bis 6 Gew.-% und vorzugsweise 1,5 bis 3 Gew.-% superabsorbierende Polymergranulate,
(c) 75 bis 95 Gew.-% und vorzugsweise 85 bis 90 Gew.-% Wasser,
(d) 6 bis 10 Gew.-% und vorzugsweise 7 bis 9 Gew.-% eines Feuchthaltemittels,
wobei die Prozentsätze als Gewichtsprozentsatz in Bezug auf das Gesamtgewicht der Zusammensetzung ausgedrückt sind und das Gesamtgewicht der Zusammensetzung 100 % ausmacht.

3. Kryogene Zusammensetzung nach Anspruch 1 oder Anspruch 2, **dadurch gekennzeichnet, dass** die hydrophobe Verbindung (a) einen Gefrierpunkt unter -15 ºC und weiterhin bevorzugt unter -20 ºC aufweist.

4. Kryogene Zusammensetzung nach einem der Ansprüche 1 bis 3, **dadurch gekennzeichnet, dass** das superabsorbierende Polymer (b) aus vernetzten Natrium-oder Kaliumpolyacrylaten, Polyacrylamiden, Copolymeren auf der Basis von Ethylen und von Maleinanhydrid, Vinylalkoholcopolymeren, vernetztem Polyethylenoxid, Polymeren auf der Basis von Stärke, von Gummi und von Zellulosederivaten, Pektinen, Alginaten, Agar-Agar (oder Agarose), Polyethylenaminen, Polyvinylaminen und deren Mischungen ausgewählt ist.

5. Kryogene Zusammensetzung nach einem der Ansprüche 1 bis 4, **dadurch gekennzeichnet, dass** die superabsorbierenden Polymergranulate (b) in Form von kugelförmigen Partikeln mit einem Durchmesser im Bereich zwischen 1 und 6 mm vorliegen, wenn sie dehydratisiert sind, wobei die kugelförmigen Partikel wenigstens das 40-fache ihrer Masse absorbieren können.

6. Kryogene Zusammensetzung nach Anspruch 5, **dadurch gekennzeichnet, dass** die kugelförmigen Partikel des superabsorbierenden Polymers (b) Kugeln aus Natrium-oder Kaliumpolyacrylat, vorteilhafterweise aus Kaliumpolyacrylat, mit einem Durchmesser im Bereich zwischen 1 und 3 mm sind, wenn sie dehydratisiert sind.

7. Kryogene Zusammensetzung nach einem der Ansprüche 1 bis 6, **dadurch gekennzeichnet, dass** das Feuchthaltemittel (d) aus Glycerin, Sorbit, Polyethylenglykol, (Di)propylenglykol, Polypropylenglykol, 1,5-Pentandiol, Propylenglykol, Butylenglykol, Diethylenglykol, Paraffinöl und deren Mischungen ausgewählt ist.

8. Kryogene Zusammensetzung nach Anspruch 7, **dadurch gekennzeichnet, dass** das Feuchthaltemittel (d) Dipropylenglykol ist.

9. Verfahren zur Herstellung einer kryogenen Zusammensetzung nach einem der Ansprüche 1 bis 8, **dadurch gekennzeichnet, dass** es die folgenden Schritte umfasst:
(i) Mischen des Wassers (c) und des Feuchthaltemittels (d) unter Rühren,
(ii) Hinzufügen der superabsorbierenden Polymergranulate (b) zu der am Ende des Schrittes (i) erhaltenen Mischung unter Rühren,
(iii) Reifen der superabsorbierenden Polymergranulate (b), die in der am Ende des Schrittes (ii) erhaltenen Mischung vorhanden ist, unter Ruhenlassen der Mischung bei Raumtemperatur, vorzugsweise bis die superabsorbierenden Polymergranulate (b) eine Größe zwischen dem Zweifachen und dem Vierfachen ihrer anfänglichen Größe erreicht haben,
(iv) Einbringen unter Rühren der am Ende des Schrittes (iii) erhaltenen Mischung in einen Behälter, der die hydrophobe Verbindung (a) in flüssiger Form umfasst.

10. Verfahren nach Anspruch 9, **dadurch gekennzeichnet, dass** der Schritt (iii) des Reifens der superabsorbierenden Polymergranulate (b) für eine Dauer im Bereich von 1 bis 5 Stunden und vorzugsweise für eine Dauer im Bereich von 2 bis 4 Stunden durchgeführt wird.

11. Vorrichtung zur Wärmebehandlung, **dadurch gekennzeichnet, dass** sie einen dichten Behälter umfasst, in dem eine kryogene Zusammensetzung wie in den Ansprüchen 1 bis 8 definiert enthalten ist.

12. Vorrichtung nach Anspruch 11, **dadurch gekennzeichnet, dass** der Behälter ein dichter und flexibler medizinischer Beutel ist, der aus einem Material besteht, das aus Polyvinylchlorid (PVC), Polychloropren (Neopren), Polytetrafluorethylen (PTFE) oder Polyethylen (PE) ausgewählt ist.

13. Medizinische Vorrichtung, ausgewählt aus einer Gesichtsmaske, einer Schiene für die Schulter, den Ellbogen, den Knöchel, das Knie, die Hüfte oder das Handgelenk und jedweder Stütze, die einen dichten und flexiblen medizinischen Beutel wie in Anspruch 12 definiert umfasst.

14. Verwendung einer kryogenen Zusammensetzung nach einem der Ansprüche 1 bis 8 oder einer Vorrichtung zur Wärmebehandlung nach Anspruch 11, um die Temperatur von Lebensmitteln zu senken oder aufrechtzuerhalten oder um deren Konservierung zu begünstigen, oder für den Transport von wärmeempfindlichen Artikeln in kalter Umgebung.

## Claims

1. A cryogenic composition, **characterized in that** it comprises a hydrophobic compound (a) in the liquid form in which granules of superabsorbent polymer (b) charged with water (c) and with humectant (d) are immersed, said hydrophobic compound (a) having a freezing point of less than - 10°C and being chosen from neopentylene glycol diheptanoate, isopropyl sebacate, isodecyl neopentanoate, isostearyl isostearate and their mixtures.

2. The cryogenic composition as claimed in claim 1, **characterized in that** it comprises:
(a) from 0.2% to 3% and preferably from 0.2% to 2% by weight of a hydrophobic compound, the freezing point of which is less than 0°C,
(b) from 1% to 6% and preferably from 1.5% to 3% by weight of superabsorbent polymer granules,
(c) from 75% to 95% and preferably from 85% to 90% by weight of water,
(d) from 6% to 10% and preferably from 7% to 9% by weight of a humectant,
said percentages being expressed as percentages by weight with respect to the total weight of the composition, and the total weight of the composition representing 100%.

3. The cryogenic composition as claimed in claim 1 or claim 2, **characterized in that** the hydrophobic compound (a) has a freezing point of less than -15°C and more preferably still of less than -20°C.

4. The cryogenic composition as claimed in one of claims 1 to 3, **characterized in that** the superabsorbent polymer (b) is chosen from crosslinked sodium or potassium polyacrylates, polyacrylamides, copolymers based on ethylene and maleic anhydride, vinyl alcohol copolymers, crosslinked polyethylene oxide, polymers based on starch, on gum and on cellulose derivatives, pectins, alginates, agar (or agarose), polyethyleneamines, polyvinylamines and their mixtures.

5. The cryogenic composition as claimed in one of claims 1 to 4, **characterized in that** said granules of superabsorbent polymer (b) are provided in the form of spherical particles having a diameter of 1 to 6 mm when they are dehydrated, said spherical particles being able to absorb at least 40 times their weight.

6. The cryogenic composition as claimed in claim 5, **characterized in that** said spherical particles of superabsorbent polymer (b) are sodium or potassium polyacrylate beads, advantageously potassium polyacrylate beads, having a diameter of 1 to 3 mm when they are dehydrated.

7. The cryogenic composition as claimed in one of claims 1 to 6, **characterized in that** the humectant (d) is chosen from glycerol, sorbitol, polyethylene glycol, (di)propylene glycol, polypropylene glycol, 1,5-pentanediol, propylene glycol, butylene glycol, diethylene glycol, liquid paraffin and their mixtures.

8. The cryogenic composition as claimed in claim 7, **characterized in that** the humectant (d) is dipropylene glycol.

9. A process for the preparation of a cryogenic composition as claimed in one of claims 1 to 8, **characterized in that** it comprises the following steps:
(i) mixing the water (c) and the humectant (d), under stirring,
(ii) adding the granules of superabsorbent polymer (b) to the mixture obtained at the end of step (i), under stirring,
(iii) maturing the granules of superabsorbent polymer (b) present within the mixture obtained at the end of step (ii), by allowing said mixture to stand at room temperature, preferably until the granules of superabsorbent polymer (b) have reached a size of between two and four times their initial size,
(iv) incorporating the mixture obtained at the end of step (iii) in a container comprising the hydrophobic compound (a) in the liquid form, under stirring.

10. The process as claimed in claim 9, **characterized in that** step (iii) of maturing the granules of superabsorbent polymer (b) is carried out over a period of time ranging from 1 to 5 hours, and preferably over a period of time ranging from 2 to 4 hours.

11. A thermal treatment device, **characterized in that** it comprises a leaktight container, within which is contained a cryogenic composition as defined in claims 1 to 8.

12. The device as claimed in claim 11, **characterized in that** said container is a leaktight and flexible medical bag made of a material chosen from polyvinyl chloride (PVC), polychloroprene (neoprene), polytetrafluoroethylene (PTFE) or polyethylene (PE).

13. A medical device chosen from a face mask, a splint for the shoulder, elbow, ankle, knee, hip or wrist, and any support comprising a leaktight and flexible medical bag as defined in claim 12.

14. The use of a cryogenic composition as defined according to one of claims 1 to 8, or of a thermal treatment device as claimed in claim 11, for lowering or maintaining the temperature of foodstuffs, or for promoting their preservation, or for the transportation in cold environment of heat-sensitive articles.
